# EUROPEAN PATENT SPECIFICATION

(11) **EP 2 629 778 B1**
(45) Date of publication and mention of the grant of the patent: **21.09.2016**
(21) Application number: 11799304.8
(22) Date of filing: 18.10.2011
(51) Int. Cl.: A61K 31/5575, A61P 1/04, A61K 9/28

(54) **USE OF MEXIPROSTIL IN THE TREATMENT OF INFLAMMATORY BOWEL DISEASE AND/OR OF IRRITABLE BOWEL SYNDROME**
VERWENDUNG VON MEXIPROSTIL BEI DER BEHANDLUNG VON CHRONISCH-ENTZÜNDLICHER DARMERKRANKUNG UND/ODER DES REIZDARMSYNDROMS
UTILISATION DE MEXIPROSTIL DANS LE TRAITEMENT D'UNE MALADIE INTESTINALE INFLAMMATOIRE ET/OU D'UN SYNDROME DU CÔLON IRRITABLE

(30) Priority: 19.10.2010 IT MI20101908
(43) Date of publication of application: 28.08.2013
(73) Proprietor: Cosmo Technologies Ltd., Dublin 2 (IE)
(72) Inventor: ASSANDRI, Alessandro, 6850 Mendrisio (CH); MORO, Luigi, I-21050 Cairate (VA) (IT)
(74) Representative: Pistolesi, Roberto
(86) International application number: PCT/IB2011/054627
(87) International publication number: WO 2012/052918

(56) References cited:
- EP-A1- 0 325 869
- WO-A1-00/76481
- US-A- 5 219 885
- US-A1- 2004 138 308
- SPINA G ET AL: "MDL-646, a new synthetic E1-prostaglandin with local protective effects on the gastric mucosa", PROSTAGLANDINS, BUTTERWORTH, STONEHAM, MA, US, vol. 28, no. 2, 1 August 1984 (1984-08-01) , pages 158-171, XP023448477, ISSN: 0090-6980, DOI: DOI:10.1016/0090-6980(84)90053-4 [retrieved on 1984-08-01]
- PETRILLO M ET AL: "Gastric antisecretory and cytoprotective activity of MDL 646, a 16-methyl-16-methoxy prostaglandin E1 analog in man", HEPATO-GASTROENTEROLOGY, THIEME, STUTTGART, DE, vol. 34, no. 3, 1 January 1987 (1987-01-01), pages 117-119, XP009146918, ISSN: 0172-6390
- VAN HIJFTE L ET AL: "Towards a large scale preparation of mexiprostil", TETRAHEDRON 1992 GB LNKD- DOI:10.1016/S0040-4020(01)88229-6, vol. 48, no. 31, 1992, pages 6393-6402, XP002631821, ISSN: 0040-4020

## Description

The present invention relates to mexiprostil for use in the treatment and/or prevention of inflammatory bowel disease (IBD) and of irritable bowel syndrome (IBS) as defined in the claims.

### Technical field

Mexiprostil is the international non-proprietary name of (8alpha,11alpha,15R,16R) 11,15-dihydroxy-16-methyl-16-methoxy-9-oxoprost-13-en-1-oic acid methyl ester, having the following formula

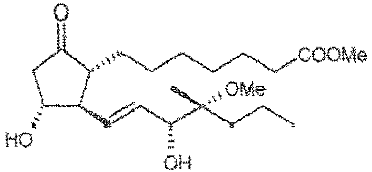

Mexiprostil is therefore a synthetic derivative of PGE1 prostaglandins which has been developed for the treatment of excessive gastric secretion and for the cytoprotection of the gastric mucosa.

The diseases of the digestive system are various. The expression "inflammatory bowel disease" or IBD indicates a series of chronic inflammatory intestinal pathologies, including principally ulcerative colitis and Crohn's disease, which should not be confused with irritable bowel syndrome or IBS which is a completely different illness which, in addition to an inflammatory or post-infective component, is also thought to be due to an abnormal function of the musculature of the gastro-intestinal tract or of the nerves which control that musculature.

The pathogenesis of IBD is not known. It is thought that there may be a genetic predisposition but also environmental factors, viruses, bacteria, an immune component, which is perhaps why food antigens may set off an enteric inflammatory cascade.

Ulcerative colitis typically exhibits a continuous inflammation which extends proximally from the rectum to include, in many subjects, the entire colon.

Unlike ulcerative colitis, Crohn's disease is located principally in the small intestine but may also involve the entire intestinal tract from the mouth to the anus, with granulomatous formations and discontinuous focal ulcerations and also with fistula formation.

At the moment there is no real cure for IBD and treatment is limited to the symptomatology by inhibition of the inflammatory phenomena in an attempt to improve the patient's quality of life. The drugs used are mainly 5-ASA (or mesalazine) and its prodrugs or derivatives, such as, for example, mesalazine and its derivatives, antibiotics, corticosteroids and immunosuppressants.

Unlike IBD, irritable bowel syndrome (IBS) does not exhibit obvious anatomical impairment of the mucosa, so that it is considered a predominantly functional disease. From a purely clinical point of view, IBS is characterized by impairment of the intestinal function with constipation or diarrhoea, which sometimes alternate, by states of abdominal pain, by abdominal distension and by meteorism. The aetiology and the pathogenesis of IBS are not known, but various factors may contribute to causing its onset. Those factors often include anomalies in the regulation of the tone and activity of the smooth musculature of the intestinal wall, the effects of irritant substances of a bacterial nature and psychological and emotional influences.

In the case of IBS, the drugs most commonly used, alone or in combination, are represented by antispastics, by antidiarrhoeals or by prokinetics, depending on the prevalence of diarrhoea or constipation, respectively, by anti-infectives, by adsorbents and by minor and major anxiolytics.

As mentioned, those drugs used in the treatment of IBD and IBS do not "cure" the disease but simply relieve the symptoms thereof.

It is therefore necessary to find a real therapy which can be used to cure IBD and IBS and which is not limited to treating the symptomatology but which treats directly the tissue damage and also the impaired intestinal motility.

### Description of the invention

Thus, the invention relates to mexiprostil for use in the treatment and/or prevention of inflammatory bowel disease (IBD) and irritable bowel syndrome (IBS), wherein the mexiprostil is formulated in gastroresistant and controlled release oral compositions or the mexiprostil is in combination with a drug selected from mesalazine and budesonide.

"Inflammatory bowel disease", also IBD hereinafter, is intended to indicate according to the present invention, chronic diseases of the intestinal system, in particular ulcerative colitis and Crohn's disease.

"Irritable bowel syndrome", also IBS hereinafter, is intended to indicate according to the present invention, a set of functional intestinal disorders, in particular diarrhoea, constipation, and abdominal pain and distension. Depending on the predominant type of intestinal motor impairment, the term IBS includes the constipation, diarrhoea or alternating variants of IBS, also known by the abbreviations IBS-C, IBS-D and IBS-A.

Surprisingly, it has now in fact been found that mexiprostil is capable of treating and/or preventing IBD and IBS; as will be discussed comprehensively and in detail hereinafter, preclinical trials have demonstrated that mexiprostil reduces macroscopic damage to the intestinal walls, the size of the damaged area and the laxative effect caused by the irritant agents used to induce the pathology in the animals subjected to the test. Thanks to those trials, which will be described in detail in the experimental section of the present description, it has been possible to show that mexiprostil is capable of treating IBD and IBS, reducing the damage and the lesions caused by the inflammation, and therefore bringing about actual healing of the damaged intestinal tissue. The efficacy demonstrated in the animal tests in fact shows that mexiprostil is also capable of influencing intestinal motility, being of particular use in the various forms of IBS.

In particular, mexiprostil was tested at various doses in a model of colitis induced by acetic acid in rats. When administered topically at a dose of 10 micrograms/kg to the mucosa of the colon, mexiprostil proved to be capable of performing a powerful antiinflammatory activity and a powerful regulatory activity in respect of intestinal motility. For the use according to the invention, mexiprostil may be administered preferably via the oral route or, alternatively, via the rectal route.

Preferably, the oral route is in the form of capsules, tablets, granules or a powder, solution or suspension while the rectal route is preferably in the form of an enema, a suppository or a rectal foam.

The posology may vary widely depending on the patient's age, weight and state of health, and also on the nature and severity of the illness. By way of example, for the use according to the invention, mexiprostil is administered via the oral route at doses of from 4 to 40 micrograms/kg, advantageously from 8 to 20 micrograms/kg body weight.

Those quantities may of course vary in accordance with the requirements and characteristics of the subject to be treated.

Mexiprostil for the use according to the invention may be formulated in pharmaceutical compositions for administration to mammals, including humans, for the treatment of the above-mentioned diseases, if desired or necessary in combination with pharmacologically acceptable vehicles and/or excipients.

The pharmaceutical compositions for the use according to the invention are advantageously presented in dosage units. Appropriate unitary forms of administration include forms via the oral route, such as tablets, capsules, powders, granules and oral solutions or suspensions, or rectal forms, such as, for example, in the form of suppositories.

Advantageously, these oral pharmaceutical compositions will be of the delayed-release type, and will preferably be gastroresistant or enterosoluble formulations, so that the active ingredient is not absorbed in the stomach but is released mainly at the site of action, in this particular case in the intestine (terminal ileum, colon). These pharmaceutical compositions are therefore advantageously intended for the treatment and/or prevention of IBD and IBS.

Such formulations are known in the art and may be constituted, for example, by traditional tablets, granules or capsules surface-coated with a layer of polymer material which is insoluble in the stomach but soluble in the intestine, or also mixed with polymers which prevent them dissolving before they reach the intestine and/or the preselected section of the intestine. Any system may be employed for the use according to the invention provided it releases the mexiprostil mainly in the intestine.

As mentioned, many formulations suitable for the purpose are known. A particularly suitable formulation for the use according to the invention is, for example, the controlled-release formulation described in the patent application WO00/76481, which is incorporated herein by reference.

Advantageously, the mexiprostil-containing formulation suitable for the purpose of the invention therefore comprises:
a) a matrix which is composed of lipophilic compounds having a melting point of less than 90°C and optionally of amphiphilic compounds in which the active ingredient is at least partially incorporated;
b) optionally an amphiphilic matrix;
c) an outer hydrophilic matrix in which the lipophilic matrix and the optional amphiphilic matrix are dispersed;
d) optionally other excipients.

According to the invention, the lipophilic matrix is preferably constituted by compounds selected from fatty acids or unsaturated or saturated alcohols, their salts, esters or amides, mono-, di- or triglyceride fatty acids, their polyoxyethylated derivatives, waxes, ceramides, cholesterol derivatives or mixtures thereof having a melting point in the range of from 40 to 90°C, preferably from 60 to 70°C.

Unsaturated or saturated fatty acids according to the invention may be, for example, palmitic acid, stearic acid, myristic acid, lauric acid, oleic acid or mixtures thereof.

The amphiphilic matrix is preferably constituted by type I or II polar lipids, such as, for example, lecithin, phosphatidylcholine, phosphatidylethanolamine, ceramides, glycol alkyl esters, such as, for example, diethylene glycol monomethyl ether (Transcutol).

The hydrophilic matrix is preferably constituted by substances known as "hydrogens", or substances which, when they pass from the dry state to the hydrated state, by means of so-called molecular relaxation, a significant increase in mass and weight following the coordination of the large number of molecules of water from the polar groups present in the polymer chains of the hydrophilic substances. Some examples of hydrogels according to the invention may be compounds selected from polymers or copolymers of acrylic or methacrylic acid, alkyl vinyl polymers, hydroxyalkylcelluloses (such as carboxyalkylcelluloses), polysaccharides, dextrins, pectins, amides and derivatives thereof, synthetic or natural gums, alginic acid.

According to one particular embodiment of the invention, the formulation containing mexiprostil may also comprise a gastroresistant outer coating film, preferably of polymers of acrylic or methacrylic acid, copolymers of acrylic or methacrylic acid or mixtures thereof (Eudragit).

The formulation according to the above-mentioned embodiment of the invention is therefore a multimatrix formulation having a macroscopically homogeneous structure (i.e. not stratified like a reservoir) along the entire axis of symmetry of the final form (see also WO00/76478).

In general, the dosage units may comprise from 0.25 to 2.5 mg of mexiprostil, advantageously from 0.5 to 1.5 mg of the said active ingredient. These dosage units may be administered one or more times per day, for example 1 or 2 times per day.

Mexiprostil can therefore be used in the treatment and/or prevention of IBD and IBS, alone or in combination with other drugs, for example, with the drugs usually used in the therapy and/or symptomatic resolution of IBD and IBS. Thus, according to another of its aspects, the invention comprises the use of mexiprostil in combination with one or more drugs normally used in the therapy and/or symptomatic resolution of IBD or IBS, for example in combination with 5-ASA (mesalazine) and derivatives, such as sulphasalazine and/or olsalazine, antibiotics, such as metronidazole, ciprofloxacin, rifamycin and/or corticosteroids, such as prednisolone, budesonide and/or immunosuppressants, such as, for example, 6-mercaptopurine.

A drug particularly suitable for combination with mexiprostil is mesalazine (also known as 5-ASA or 5-aminosalicylic acid).

Another drug particularly suitable for combination with mexiprostil is budesonide.

According to one of its embodiments, the invention therefore relates to mexiprostil for use in the treatment and/or prevention of IBD, in particular ulcerative colitis and Crohn's disease, and also for use in the treatment and/or prevention of IBS, in particular IBS-C and/or IBS-D and/or IBS-A.

According to another of its embodiments, the invention relates to a combination of mexiprostil and a drug selected from mesalazine and budesonide, for use in the treatment and/or prevention of IBD, in particular ulcerative colitis and Crohn's disease, and also for use in the treatment and/or prevention of IBS.

According to a preferred embodiment, the combination of mexiprostil and a drug selected from mesalazine and budesonide is formulated in a gastroresistant and controlled-release manner, for example as described in the patent application WO00/76481, which is herein referred to.

The pharmaceutical compositions comprising the active ingredient mexiprostil, optionally in combination with pharmaceutically acceptable excipients and/or vehicles, constitute a further subject of the invention.

The pharmaceutical compositions comprising mexiprostil and mesalazine as active ingredients, optionally in combination with pharmaceutically acceptable excipients and/or vehicles, constitute a further subject of the invention.

The pharmaceutical compositions comprising mexiprostil and budesonide as active ingredients, optionally in combination with pharmaceutically acceptable excipients and/or vehicles, constitute a further subject of the invention.

Advantageously, the above-mentioned pharmaceutical compositions of the invention containing the active ingredient mexiprostil as the only active ingredient or in combination with mesalazine or budesonide and optional pharmaceutically acceptable excipients and/or vehicles, are advantageously formulated in optionally gastroresistant controlled-release manner, for example, as described in the patent application WO00/76481, which is herein referred to.

In particular, the said pharmaceutical compositions containing mexiprostil according to the invention are homogeneous multimatrix compositions containing matrices as described above, and/or:
e) a matrix which is composed of lipophilic compounds having a melting point of less than 90°C and optionally of amphiphilic compounds in which the active ingredient is at least partially incorporated;
f) optionally an amphiphilic matrix;
g) an outer hydrophilic matrix in which the lipophilic matrix and the optional amphiphilic matrix are dispersed;
h) optionally other excipients.

The lipophilic, amphiphilic and hydrophilic matrices and the optional gastroresistant coating film according to the invention refer to the compounds indicated and described above.

According to another of its aspects, the disclosure relates to a method for the treatment of IBD and/or IBS which comprises administering to a subject requiring it, an efficacious amount of mexiprostil alone or in combination with one or more drugs usually used in the therapy and/or symptomatic resolution of IBD and/or IBS as indicated above; for example, mesalazine, its derivatives, or budesonide.

Preferably, the method of the present disclosure comprises the administration of a pharmaceutical form having controlled release such as to be capable of releasing the active ingredient in the intestine, for example as described in WO00/76481.

According to this aspect of the disclosure, the method comprises the administration of a homogeneous multimatrix formulation such as described above, containing matrices such as described above

Mexiprostil is a compound known in the art, and various methods for the preparation thereof are known.

According to another of its aspects, the disclosure relates to a method for the preparation of mexiprostil as defined in Scheme 1:

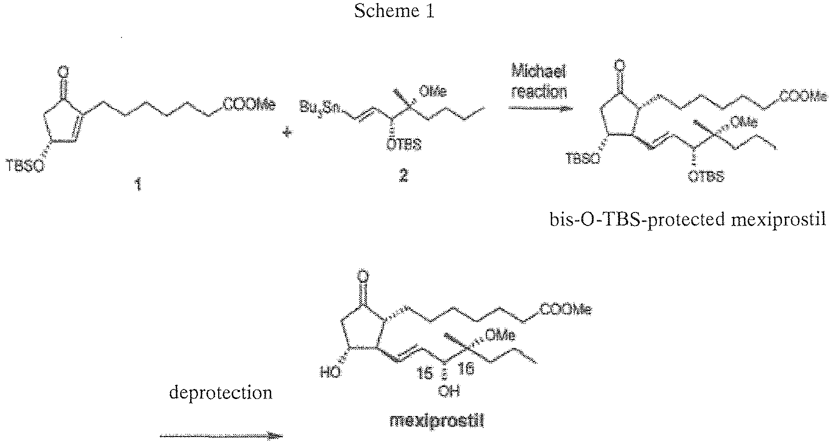

The Michael reaction of Scheme 1 was found to be particularly complicated since the various attempts made using the methods known in the art for this reaction were unsuccessful. A novel and original method was therefore found comprising the in-situ preparation of the compound of formula 2 starting from the corresponding vinyl derivative, in the presence of butyllithium and copper(I) pent-1-ynyl (CuCCPr), and its subsequent reaction with the compound of formula 1 to give protected mexiprostil which can then be deprotected in accordance with known methods, advantageously with hydrofluoric acid in an organic solvent.

The starting compounds used in Scheme 1 were obtained as follows.
Compound 1 was obtained in accordance with the following scheme: 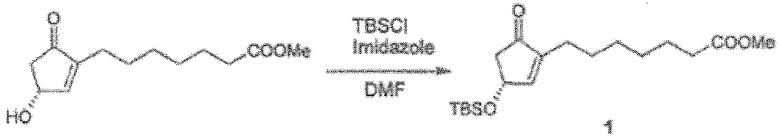 by reacting commercial (R)-4-hydroxy-2-(6-methoxycarbonylhexyl)-cyclopent-2-enone with TBSCl as described in the experimental section of the present description.
Compound 2 was obtained in accordance with the following Scheme 2: Scheme 2

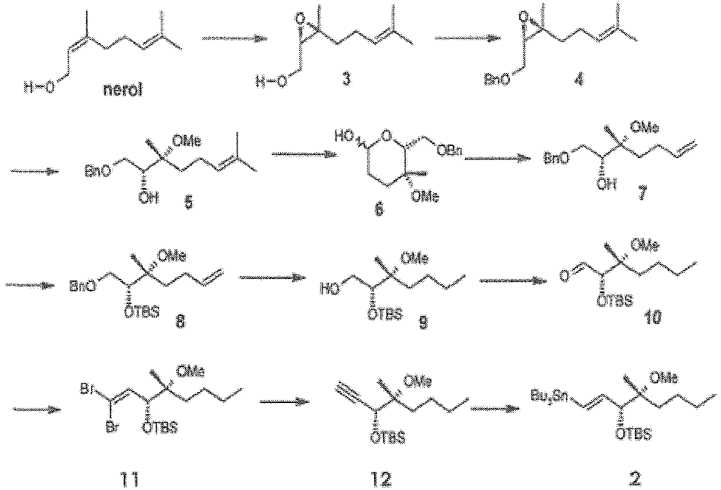

The following Examples are intended to describe the invention more clearly without in any way limiting it.

### Experimental Section

### Example 1

### General procedure:

The animals were fasted with free access to water for two days before inducing colitis. 7- to 8-week-old male rats, under light anaesthesia, were administered a single intracolonic dose of 1 mL of 4 % acetic acid in water through a catheter (day 1). Thirty minutes after inducing colitis, the rats were treated with a single intracolonic dose of the test compound or of a vehicle (saline) for control purposes. On the second day of the experiment, the rats were sacrificed by inhaling CO₂ and subjected to an autopsy. The colon was isolated and 10-cm segments of the distal portion of the colon were cut off and weighed. The Mucosal Damage Area (MDA) and the Macroscopic Damage Score (MDS) were evaluated. The histological evaluation was carried out as follows: after having weighed the colon, an intermediate portion of open isolated distal colon measuring approximately 2 cm was fixed in a 10% buffered formalin solution, and then cut transversely and stained with H&E stain and PAS stain. Myeloperoxidase activity was measured on the remaining portions of the colon.

### Example 2

### Evaluation

The administration of 4% acetic acid in accordance with an experimental model which was considered to be indicated for the treatment of IBD and IBS, caused obvious macroscopic damage, increased the weight of the colon, inhibited body growth and induced diarrhoea.

There were no deaths in the two groups. The administration of mexiprostil at a dose of 10 micrograms/kg provided a significant improvement in the MDS (-77.2%) and the MDA (-66%), a reduction in the weight of the colon and an increase in body weight and substantially reduced the diarrhoea (-40.8%). At the same dose, a significant inhibition of the myeloperoxidase activity in the colon was observed (- 39.9%).

### Example 3

### Preparation of TBS-protected (R)-4-hydroxy-2-(6-methoxycarbonylhexyl)-cyclopenta- 2-enone (compound 1, Scheme 1)

650 mg (98.2% e.e.) of (R)-4-hydroxy-2-(6-methoxycarbonylhexyl)-cyclopenta-2-enone were dissolved in dimethylformamide (DMF) (13.4 mL), and then 384.4 mg of imidazole (5.65 mmols) and 815.7 mg of TBSCl (5.41 mmols) were added. The reaction was agitated overnight and stopped by the addition of water and diethyl ether. The layers were separated and the aqueous phase was extracted three times with diethyl ether. The combined organic extracts were washed with water, saline and dried over sodium sulphate. The solvent was evaporated and the crude product was purified by column chromatography (hexane/acetic acid 96/4) to give compound 1 in pure form (938.5 mg, 98% yield).

### Example 4

### Preparation of bis-O-TBS-protected mexiprostil

### (Preparation of compound 2 in situ and subsequent reaction with compound 1, Example 3)

7 mg of PdCl₂(PPh₃)₂ (0.01 mmol) were added under an argon atmosphere to an agitated solution of the compound of formula 12 (Scheme 2, Example 6 below) (240 mg, 0.84 mmol) in 5 mL of anhydrous THF. 250 microL (0.93 mmol) of n-Bu₂SnH were then added and the reaction was left under agitation for 15 minutes (check with TLC that the starting alkyne had been consumed). The reaction mixture was cooled to -60°C and three cycles of vacuum under argon were effected. 1.1 mL of a 1.6M solution in hexane of n-BuLi (1.76 mmols) were added dropwise and the reaction mixture was agitated for 10 minutes (check with TLC that the vinyl stannane had been consumed). A further three cycles of vacuum under argon were effected. A solution of copper pentino²-HMPT complex (copper(1) pent-1-ynyl; CuCCPr) was added to the reaction mixture at -60°C, the said solution being obtained by adding HMPT (552 mg, 3.38 mmols) to a suspension of copper pentino² (220 mg, 1.69 mmols) in anhydrous THF at ambient temperature. After 20 minutes, compound 1 (150 mg, 0.42 mmol) dissolved in 2 mL of THF was added to the reaction mixture and the whole was agitated for 30 minutes. The reaction was stopped by adding a saturated solution of NR₄Cl and dilution was effected with ethyl acetate. The layers were separated and the aqueous phase was extracted three times with ethyl acetate. The combined organic extracts were dried over sodium sulphate and the solvent was removed under vacuum. The crude reaction mixture was purified by column chromatography (hexane/ethyl acetate 8/2) to obtain the title compound (127 mg).

### Example 5

### Preparation of mexiprostil

48% hydrofluoric acid (300 microL, 7.72 mmols) was added to a solution of the compound of Example 4 (127 mg, 0.193 mmol) in 20 mL of acetonitrite. The reaction was agitated for 36 hours and then stopped with phosphate buffer (pH=6.8) and diluted with ethyl acetate. The layers were separated and the aqueous phase was extracted three times with ethyl acetate. The combined organic extracts were dried over sodium sulphate and the solvent was removed under vacuum. The crude reaction mixture was purified by column chromatography (ethyl acetate 100%) to give pure mexiprostil (40 mg). The ¹H and 13C NMR spectra (CDCl₃, 300 MHz) were in accordance with those reported in the literature.

### Example 6

### Preparation of the compound of formula 12 (Scheme 2)

### Example 6.1 - Compound 3 Scheme 2 - [3-(2R, 3S)-2,3-epoxy-3,7-dimethyl-6-octen-1-ol]

Anhydrous DCM (methylene chloride) (280 mL) was added to a flask containing active molecular sieves (4A, 10 g) in an argon atmosphere. The reaction mixture was cooled to -15°C and then 21.7 g, 105.2 mmols of (-)-DET ((-)-diethyl tartrate) were added, followed by titanium isopropylate (24.3 mL, 81.7 mmols) and t-butyl hydroperoxide (35 mL, 210 mmols, solution in nonane 5.0-6.0M). The reaction mixture was agitated at -15°C for 30 minutes and was then cooled to -20°C and nerol (28.0 g, 181.5 mmols) was added dropwise, avoiding an increase in temperature. After three hours at -20°C, the starting material is completely consumed (TLC analysis, eluant hexane/ethyl acetate 8/2). The cooling bath was removed and the mixture was left to return to 0°C. At that point, water (800 mL) was added in a single portion under vigorous agitation. After 15 minutes, an aqueous solution of NaOH and NaCl (12 g NaCl dissolved in 225 mL of NaOH 8M) was added. The two phases together with the emulsion formed were transferred into a separating funnel. The layers were separated and the aqueous phase was extracted 5 times with diethyl ether. The combined organic extracts were dried over sodium sulphate and the solvent was removed under vacuum to give the crude compound 3 which was purified on a chromatographic column (hexane/EtOAc 8/2) to give the pure compound 3 (21 g, 68%).

### Example 6.2

### Compound 4 Scheme - (2R, 3S)-1-benzyloxy-2,3-epoxy-3, 7-dimethyl-6-octene

Compound 3 (17.2 g, 101.0 mmols) was dissolved in anhydrous THF (110 mL) in an argon atmosphere and the mixture was cooled to -10°C. Benzyl bromide (19.0 g, 111.1 mmols) and KOtBu (12.5 g, 111.1 mmols) were added. The cooling bath was removed and the reaction was agitated overnight. The reaction mixture was divided between diethyl ether and a saturated solution of NH₄Cl. The layers were separated and the aqueous phase was extracted 3 times with diethyl ether. The combined organic extracts were dried over sodium sulphate and the solvent was removed under vacuum. The crude compound 4 (26.2 g) was used without further purification in the next step.

### Example 6.3

### Compound 4 Scheme 2 - (2R, 3S)-1-benzyloxy-3-methoxy-3, 7-dimethyl-6-octen-1-ol

Compound 4 (26.2 g, 100.6 mmols) was dissolved in MeOH (210 mL), and Dowex 50 (1.3 g) was added. The reaction was agitated overnight and the resin was filtered and washed with DCM. The solvent was evaporated under vacuum and the crude product was purified by column chromatography (hexane/EtOAc 95/5 -> 90/10 -> 80/20) to give the pure compound 5 (23.8 g).

### Example 6.4

### Compound 6 Scheme 2

Compound 5 (23.7 g, 81.2 mmols) was dissolved in DCM/MeOH 1/1 (mL) and the resultant solution was cooled to -78°C. O₃ was bubbled through until the mixture turned light blue. Two drops of Me₂S were then added and then N₂ was bubbled through. PPh₃ (21.78 g, 83.0 mmols) was then added and the reaction mixture was agitated for 90 minutes while the temperature rose to ambient temperature again. The solvent was removed under vacuum. The crude reaction mixture was dissolved in THF (37 mL) and HCl 2.4M (22 mL) and agitated until the more polar spots in the TLC (caused by the metal-acetal of the desired lactol 6, by-product) disappeared (normally 4-5 days). When the reaction appeared to stop, more HCl (in solution at 37%) was added (eluant for TLC analysis: hexane/EtOAc 6/4). The reaction was stopped by adding saline and diethyl ether. The two layers were separated and the organic phase was extracted with saline. The combined organic phases were washed with a solution of NaHCO₃ followed by saline and then dried over sodium sulphate and purified by chromatography (hexane/EtOAc 1/1) to give compound 6 (16 g) as a mixture of diastereoisomers.

### Example 6.5

### Compound 7 Scheme 2 - (2R, 3R)-1-benzyloxy-3-methoxy-3-methyl-6-heptan-2-ol)

Ph₃PCH₃Br (35.3 g, 98.9 mmols) was suspended in anhydrous THF (101 mL) and cooled in an ice-bath and then KOtBu (14.8 g, 131.8 mmols) was added dropwise. The cooling bath was removed and the yellow solution formed was agitated for 30 minutes. Compound 6 (11.7 g, 43.9 mmols) dissolved in THF (46 mL) was then added. The reaction was agitated for 3.5 hours and then poured into a saturated solution of NaCl and EtOAc. The layers were separated and the aqueous phase was extracted 3 times with ethyl acetate. The combined organic extracts were dried over sodium sulphate and the solvent was removed under vacuum. The crude compound was purified on a chromatographic column (hexane/EtOAc 9/1) to give the pure compound 7 (10.8 g).

### Example 6.6

### Compound 8

Compound 7 (10.8 g, 40.9 mmols) was dissolved in DMF (102 mL), and then imidazole (5.57 g, 81.9 mmols) and TBSCl (12.35 g, 81.9 mmols) were added. After 2 days, the reaction was stopped by adding water and diethyl ether. The layers were separated and the aqueous phase was extracted 3 times with diethyl ether. The combined organic extracts were dried over sodium sulphate and the solvent was removed under vacuum to give the crude compound 8 (15 g) which was used without further purification in the next step.

### Example 6.7

### Compound 9

Compound 8 (crude 15 g) was dissolved in EtOAc (150 mL), Pd(OH)₂ (1.5 g) was added and the mixture was agitated overnight in a hydrogen atmosphere. The catalyst was removed by filtration and the solvent was removed under vacuum to give the crude compound 9 (18 g) which was used without further purification in the next step.

### Example 6.8

### Compound 10

Compound 9 (crude 18 g) was dissolved in DMSO (250 mL) and then IBX (22.57 g) was added. After 4 hours of agitation, the reaction mixture was poured into water. The white precipitate was filtered off and washed with water and hexane. The filtration liquid was separated off and the aqueous phase was extracted 3 times with diethyl ether. The combined organic extracts were dried over sodium sulphate and the solvent was removed under vacuum to give the crude compound 10 (14.6 g).

### Example 6.9

### Compound 11

CBr₄ (31.86 g, 96.1 mmols) was dissolved in anhydrous DCM (76 mL) in an argon atmosphere and cooled in an ice-bath. Triphenylphosphine (50.9 g, 194.1 mmols) was added in portions and after 10 minutes the ice-bath was removed and the reaction mixture was left to return to ambient temperature. A solution of compound 10 (14.0 g, 48.5 mmols) in anhydrous DCM (31 mL) was then added. The reaction mixture was agitated for 4 hours and then divided between ethyl acetate and water. The aqueous phase was extracted 3 times with ethyl acetate. The combined organic extracts were washed with 1.2M HCl, a saturated solution of NaHCO₃, saline and then dried over sodium sulphate. The solvent was removed and the solid obtained was suspended in ethyl acetate, filtered and washed with a large amount of ethyl acetate. The solvent was evaporated and the crude product was purified by column chromatography (hexane/EtOAc 99/1) to give the pure compound 11 (9.98 g).

### Example 6.10

### Compound 12

Compound 11 (9.9 g, 22.3 mmols) was dissolved in anhydrous THF (108 mL) in an argon atmosphere and the resultant solution was cooled to -78°C. n-BuLi (27 mL, 67.5 mmols, as a solution in hexane 2.5M) was added dropwise. The reaction was stopped by adding a saturated solution of NH₄Cl and diethyl ether. The layers were separated and the aqueous phase was extracted 3 times with diethyl ether. The combined organic extracts were dried over sodium sulphate and the solvent was removed under vacuum to give the crude compound which was purified by column chromotography to give the pure compound 12 (6.15 g).

### Example 7. Formulations containing mexiprostil

### Example 7.1

5 g of mexiprostil were mixed for 10 minutes with 45 g of trehalose, 2 g of lecithin, 2 g of stearic acid and 80 g of microcrystalline cellulose. 100 g of hypromellose, a further 80 g of microcrystalline cellulose, 3 g of colloidal silica and 3 g of sodium stearyl fumarate were added to the mixture and mixed in as a lubricant for the tableting operation. After further mixing, the powder was subjected to compression in a rotary tableting machine at a unit weight of 160 mg/cpr. The tablets were then coated with a gastroresistant film composed of copolymers of acrylic and methacrylic acid of type A and B, talc, triethyl citrate, titanium dioxide and iron oxides. The gastroresistant film was applied at a rate of 20 mg/cpr.

The tablets so obtained were capable of resistance for two hours in the dissolution test according to Eur. Pharm. using a medium at pH 1. In a medium at pH 7, the tablet exhibited total disintegration in approximately 8 hours using the same apparatus.

Tablets so formulated may be used in the treatment of IBD and IBS since they are intended to deliver the active ingredient principally in the colon.

### Example 7.2

5 g of mexiprostil were mixed for 10 minutes with 45 g of trehalose, 5 g of lecithin, 5 g of stearic acid and 280 g of microcrystalline cellulose. 20 g of hypromellose, a further 280 g of microcrystalline cellulose, 5 g of colloidal silica and 5 g of sodium stearyl fumarate were added to the mixture and mixed in as a lubricant for the tableting operation. After further mixing, the powder was subjected to compression in a rotary tableting machine at a unit weight of 130 mg/cpr. The tablets were then coated with a gastroresistant film composed of copolymers of acrylic and methacrylic acid of type A, talc, triethyl citrate, titanium dioxide and iron oxides. The gastroresistant film was applied at a rate of 15 mg/cpr.

The tablets so obtained were capable of resistance for two hours in the dissolution test according to Eur. Pharm. using a medium at pH 1. In a medium at pH 6.4, the tablet exhibited total disintegration in approximately 2 hours using the same apparatus.

Tablets so formulated may be used in the treatment of IBD and IBS, since they are intended to deliver the active ingredient in the small intestine.

### Example 7.3

5 g of mexiprostil were mixed for 10 minutes with 45 g of trehalose, 10 g of lecithin, 5 g of stearic acid and 385 g of microcrystalline cellulose. The mixture was kneaded and granulated using 100 g of a 5% aqueous solution of polyvinylpyrrolidone and dried; 50 g of hypromellose, a further 480 g of microcrystalline cellulose, 10 g of colloidal silica and 5 g of sodium stearyl fumarate were then added and mixed in as a lubricant for the tableting operation. After further mixing, the powder was subjected to compression in a rotary tableting machine at a unit weight of 100 mg/cpr. The tablets were then coated with a film based on hydroxypropylmethylcellulose to facilitate swallowing, at a rate of 10 mg/cpr.

The tablets so obtained dissolved within 45 minutes in the disintegration test according to Eur. Pharm. using a medium at pH 1.

### Example 7.4

5 g of mexiprostil were mixed for 10 minutes with 45 g of trehalose, 5000 g of mesalazine, 20 g of lecithin, 20 g of stearic acid and 1000 g of microcrystalline cellulose. 1000 g of hypromellose, 20 g of colloidal silica and 20 g of sodium stearyl fumarate were added to the mixture and mixed in as a lubricant for the tableting operation. After further mixing, the powder was subjected to compression in a rotary tableting machine at a unit weight of 700 mg/cpr. The tablets were then coated with a gastroresistant film composed of copolymers of acrylic and methacrylic acid of type A and B, talc, triethyl citrate, titanium dioxide and iron oxides. The gastroresistant film was applied at a rate of 30 mg/cpr and enabled the tablets to resist dissolution for 2 hours, using an apparatus according to Ph. Eur., in an acid medium at pH 1.

## Claims

1. Mexiprostil for use in the treatment and/or prevention of inflammatory bowel disease (IBD) and/or of irritable bowel syndrome (IBS), **characterized in that** it is formulated in gastroresistant and controlled-release oral compositions.

2. Mexiprostil according to claim 1, for use in the treatment and/or prevention of ulcerative colitis.

3. Mexiprostil according to claim 1, for use in the treatment and/or prevention of Crohn's disease.

4. Mexiprostil for use in the treatment and/or prevention of the constipation (IBS-C), diarrhoeal (IBS-D) or alternating (IBS-A) variants of irritable bowel syndrome (IBS).

5. Mexiprostil for use according to any one of claims 1 to 4, **characterized in that** it is administered at a rate of from 0.25 to 2.5 mg per day.

6. Mexiprostil for use according to any one of claims 1 to 5, **characterized in that** it is administered in dosage units comprising from 0.5 to 1.5 mg of mexiprostil, one or more times per day.

7. Mexiprostil for use according to any one of claims 1 to 6, **characterized in that** it is formulated in compositions which comprise:
a) a matrix which is composed of lipophilic compounds having a melting point of less than 90°C and optionally of amphiphilic compounds in which the active ingredient is at least partially incorporated;
b) optionally an amphiphilic matrix;
c) an outer hydrophilic matrix in which the lipophilic matrix and the optional amphiphilic matrix are dispersed;
d) optionally other excipients.

8. Mexiprostil for use according to claim 7, **characterized in that** the composition comprises a gastroresistant coating.

9. Combination of mexiprostil with a drug selected from mesalazine and budesonide, for use in the treatment and/or prevention of inflammatory bowel disease (IBD) or irritable bowel syndrome (IBS).

## Patentansprüche

1. Mexiprostil zur Verwendung bei der Behandlung und/oder Prävention von chronisch-entzündlicher Darmerkrankung (IBD) und/oder des Reizdarmsyndroms (IBS), **dadurch gekennzeichnet, dass** es in oralen magensaftresistenten Zusammensetzungen mit kontrollierter Freisetzung formuliert ist.

2. Mexiprostil gemäß Anspruch 1 zur Verwendung bei der Behandlung und/oder Prävention von Colitis ulcerosa.

3. Mexiprostil gemäß Anspruch 1 zur Verwendung bei der Behandlung und/oder Prävention von Crohn'scher Erkrankung.

4. Mexiprostil zur Verwendung bei der Behandlung und/oder Prävention der Verstopfungs- (IBS-C), Durchfall- (IBS-D) oder abwechselnden (IBS-A) Formen des Reizdarmsyndroms (IBS).

5. Mexiprostil zur Verwendung gemäß irgendeinem der Ansprüche 1 bis 4, **dadurch gekennzeichnet, dass** es in einer Rate von 0,25 bis 2,5 mg pro Tag verabreicht wird.

6. Mexiprostil zur Verwendung gemäß irgendeinem der Ansprüche 1 bis 5, **dadurch gekennzeichnet, dass** es in Dosierungseinheiten, welche von 0,5 bis 1,5 mg Mexiprostil enthalten, ein oder mehrmals pro Tag verabreicht wird.

7. Mexiprostil zur Verwendung gemäß irgendeinem der Ansprüche 1 bis 6, **dadurch gekennzeichnet, dass** es in Zusammensetzungen formuliert ist, welche:
a) eine Matrix zusammengesetzt aus lipophilen Verbindungen mit einem Schmelzpunkt von weniger als 90°C und optional amphiphilen Verbindungen, in welchen der aktive Inhaltsstoff zumindest teilweise inkorporiert ist;
b) optional eine amphiphile Matrix;
c) eine äußere hydrophile Matrix, in welcher die lipophile Matrix und die optionale amphiphile Matrix dispergiert sind;
d) optional andere Hilfsstoffe,
umfassen.

8. Mexiprostil zur Verwendung gemäß Anspruch 7, **dadurch gekennzeichnet, dass** die Zusammensetzung eine magensaftresistente Beschichtung umfasst.

9. Kombination von Mexiprostil mit einem Wirkstoff ausgewählt aus Mesalazin und Budesonid zur Verwendung bei der Behandlung und/oder Prävention von chronisch-entzündlicher Darmerkrankung (IBD) oder des Reizdarmsyndroms (IBS).

## Revendications

1. Mexiprostil pour utilisation dans le traitement et/ou la prévention d'une maladie intestinale inflammatoire (IBD) et/ou d'un syndrome du côlon irritable (IBS), **caractérisé en ce qu'**il est formulé dans des compositions orales gastrorésistantes et à libération contrôlée.

2. Mexiprostil selon la revendication 1, pour utilisation dans le traitement et/ou la prévention de la recto-colite hémorragique.

3. Mexiprostil selon la revendication 1, pour utilisation dans le traitement et/ou la prévention de la maladie de Crohn.

4. Mexiprostil pour utilisation dans le traitement et/ou la prévention des variantes avec constipation (IBS-C), diarrhées (IBS-D) ou alternances de constipation et de diarrhées (IBS-A) d'un syndrome du côlon irritable (IBS).

5. Mexiprostil pour utilisation selon l'une quelconque des revendications 1 à 4, **caractérisé en ce qu'**il est administré à une posologie de 0,25 à 2,5 mg par jour.

6. Mexiprostil pour utilisation selon l'une quelconque des revendications 1 à 5, **caractérisé en ce qu'**il est administré dans des formes posologiques comprenant de 0,5 à 1,5 mg de mexiprostil, une ou plusieurs fois par jour.

7. Mexiprostil pour utilisation selon l'une quelconque des revendications 1 à 6, **caractérisé en ce qu'**il est formulé dans des compositions qui comprennent :
a) une matrice qui est composée de composés lipophiles ayant un point de fusion inférieur à 90 °C et facultativement de composés amphiphiles dans lesquels la substance active est au moins partiellement incorporée ;
b) facultativement une matrice amphiphile ;
c) une matrice hydrophile externe dans laquelle la matrice lipophile et la matrice amphiphile facultative sont dispersées ;
d) facultativement d'autres excipients.

8. Mexiprostil pour utilisation selon la revendication 7, **caractérisé en ce que** la composition comprend un enrobage gastrorésistant.

9. Combinaison de mexiprostil et d'un médicament choisi parmi la mésalazine et le budésonide, pour utilisation dans le traitement et/ou la prévention d'une maladie intestinale inflammatoire (IBD) ou d'un syndrome du côlon irritable (IBS).
